# EUROPÄISCHE PATENTSCHRIFT

(11) **EP 2 081 914 B1**
(45) Veröffentlichungstag und Bekanntmachung des Hinweises auf die Patenterteilung: **02.03.2011**
(21) Anmeldenummer: 07821246.1
(22) Anmeldetag: 12.10.2007
(51) Int. Cl.: C07D 239/54, A01N 43/48

(54) **KRISTALLINE FORM VON 2-CHLOR-5-[3,6-DIHYDRO-3-METHYL-2,6-DIOXO-4-(TRIFLUORMETHYL)-1-(2H)-PYRIMIDINYL]-4-FLUOR-N-[[METHYL-(1-METHYLETHYL)AMINO]SULFONYL]BENZAMID**
CRYSTALLINE FORM OF 2-CHLORO-5-[3,6-DIHYDRO-3-METHYL-2,6-DIOXO-4-(TRIFLUOROMETHYL)-1-(2H)-PYRIMIDINYL]-4-FLUORO-N-[[METHYL-(1-METHYL-ETHYL)AMINO]SULPHONYL]BENZAMIDE
FORME CRISTALLINE DE 2-CHLORO-5-[3,6-DIHYDRO-3-METHYL-2,6-DIOXO-4-(TRIFLUOROMETHYL)-1-(2H)-PYRIMIDINYL]-4-FLUORO-N-[[METHYL-(1-METHYLETHYL)AMINO]SULFONYL]BENZAMIDE

(30) Priorität: 13.10.2006 EP 06122265
(43) Veröffentlichungstag der Anmeldung: 29.07.2009
(73) Patentinhaber: BASF SE, 67056 Ludwigshafen (DE)
(72) Erfinder: SCHMIDT, Thomas, 67433 Neustadt (DE); GEBHARDT, Joachim, 67157 Wachenheim (DE); LÖHR, Sandra, 67059 Ludwigshafen (DE); KEIL, Michael, 67251 Freinsheim (DE); WEVERS, Jan Hendrik, 67591 Hohensülzen (DE); ERK, Peter, 67227 Frankenthal (DE); SAXELL, Heidi Emilia, 67071 Ludwigshafen (DE); HAMPRECHT, Gerhard, 69469 Weinheim (DE); SEITZ, Werner, 68723 Plankstadt (DE); MAYER, Guido, 67161 Gönnheim (DE); WOLF, Bernd, 67136 Fussgönheim (DE); COX, Gerhard, 67098 Bad Dürkheim (DE); MICHEL, Alfred, 67305 Ramsen (DE); ZAGAR, Cyrill, Hong Kong (CN); REINHARD, Robert, 67117 Limburgerhof (DE); SIEVERNICH, Bernd, 67454 Hassloch (DE)
(86) Internationale Anmeldenummer: PCT/EP2007/060879
(87) Internationale Veröffentlichungsnummer: WO 2008/043835

(56) Entgegenhaltungen:
- WO-A-01/83459
- WO-A-03/097589
- WO-A-2005/054208
- WO-A-2006/010474

## Beschreibung

Die vorliegende Erfindung betrifft eine kristalline Form von 2-Chlor-5-[3,6-dihydro-3-methyl-2,6-dioxo-4-(trifluormethyl)-1-(2H)-pyrimidinyl]-4-fluor-N-[[methyl-(1 -methylethyl)amino]sulfonyl]benzamid, das im Folgenden auch als Phenyluracil I bezeichnet wird. Die Erfindung betrifft auch ein Verfahren zur Herstellung dieser kristallinen Form sowie Formulierungen für den Pflanzenschutz, welche diese kristalline Form des Phenyluracils enthalten.

Bei dem Phenyluracil I, welches der folgenden Formel entspricht, 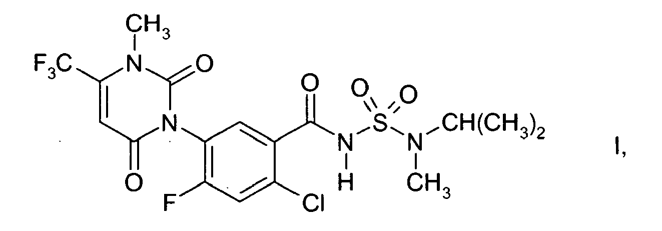 handelt es sich um einen herbiziden Wirkstoff, welcher aus der WO 01/083459 bekannt ist. Weitere Verfahren zu seiner Herstellung sind aus WO 03/097589, WO 05/054208 und WO 06/097589 sowie aus der älteren internationalen Anmeldung PCT/EP 2006/062414 bekannt. Alle bekannten Verfahren zur Herstellung des Phenyluracils I, liefern dieses als eine amorphe Substanz.

Eigene Untersuchungen der Anmelderin haben gezeigt, dass das amorphe Phenyluracil I nur bedingt zur Herstellung von Formulierungen, welche die Substanz als Feststoff enthalten, geeignet ist. Insbesondere bei mehrphasigen Formulierungen können Stabilitätsprobleme auftreten.

Es wurde nunmehr überraschenderweise gefunden, dass durch geeignete Verfahren eine kristalline, im Wesentlichen lösungsmittelfreie Form des Phenyluracils I erhalten wird, welche diese Nachteile nicht aufweist. Zudem hat sich überraschenderweise gezeigt, dass diese kristalline Form eine bessere herbizide Wirksamkeit und in einer Reihe von Kulturen eine bessere Nutzpflanzenverträglichkeit aufweist als die bislang bekannte amorphe Form des Phenyluracils I. Zudem ist die erfindungsgemäße kristalline Form kompakter als die bislang amorphe Form und fällt bei der Herstellung in Form diskreter Kristalle oder Kristallite an. Sie ist daher leichter zu handhaben als Form I. Dementsprechend betrifft die vorliegende Erfindung eine im Wesentlichen lösungsmittelfreie kristalline Form von 2-Chlor-5-[3,6-dihydro-3-methyl-2,6-dioxo-4-(trifluormethyl)-1-(2H)-pyrimidinyl]-4-fluor-N-[[methyl-(1-methylethyl)amino]sulfonyl]benzamid.

Zur Unterscheidung von der bekannten amorphen Form, im Folgenden als Form I bezeichnet, wird die erfindungsgemäße, im Wesentlichen lösungsmittelfreie Form des Phenyluracils I im Folgenden auch als Form II bezeichnet.

Bezogen auf die Form II bedeutet der Ausdruck "im Wesentlichen lösungsmittelfrei", dass die erfindungsgemäße Form II keine nachweisbaren Mengen an Lösungsmitteln im Kristallgitter eingebaut enthält, d. h. der Anteil an Lösungsmittel im Kristallgitter liegt unterhalb 10 mol-%, insbesondere nicht mehr als 5 mol-%, bezogen auf das Phenyluracil I.

Die erfindungsgemäße Form II kann mittels Röntgenpulverdiffraktometrie anhand ihres Beugungsdiagramms identifiziert werden. So zeigt ein bei 25 °C unter Verwendung von Cu-Kα-Strahlung (1,54178 Ä) aufgenommenes Röntgenpulverdiffraktogramm wenigstens 2, in der Regel wenigstens 4, häufig wenigstens 6, insbesondere wenigstens 8 und speziell alle der in der folgenden Tabelle 1 als 2θ-Werte, bzw. als Netzebenenabstände d, angegebenen Reflexe:

**Tabelle 1:**

| 2θ | d [Å] |
|---|---|
| 6,3 ± 0,2° | 14,92 ± 0,3 |
| 9,4 ± 0,2° | 9,37 ± 0,2 |
| 10,9 ± 0,2° | 8,15 ± 0,1 |
| 11,9 ± 0,2° | 7,45 ± 0,05 |
| 12,6 ± 0,2° | 7,02 ± 0,05 |
| 15,0 ± 0,2° | 5,90 ± 0,05 |
| 15,8 ± 0,2° | 5,62 ± 0,04 |
| 17,1 ± 0,2° | 5,19 ±.0,03 |
| 20,0 ± 0,2° | 4,44 ± 0,02 |
| 20,4 ± 0,2° | 4,36 ± 0,02 |
| 24,7 ± 0,2° | 3,61 ± 0,02 |
| 25,2 ± 0,2° | 3,53 ± 0,02 |
| 26,2 ± 0,2° | 3,40 ± 0,02 |

Untersuchungen an Einkristallen der Form II bei -170 °C zeigen, dass die zugrunde liegende Kristallstruktur monoklin ist. Die Elementarzelle weist die Raumgruppe P2(1)/c auf. Die charakteristischen Daten der Kristallstruktur von Form II sind in der Tabelle 2 zusa mmengefasst.

**Tabelle 2: Kristallographische Eigenschaften der Form II (gemessen bei -170 °C)**

| Parameter | Form II |
|---|---|
| Klasse | Monoklin |
| Raumgruppe | P2(1)/c |
| a | 9,377(5) Å |
| b | 7,698(4) Å |
| c | 28,12(2) Å |
| α | 90° |
| β | 96,37(3)° |
| γ | 90° |
| Volumen | 2017,1 (17) Å 3 |
| Z | 4 |
| Dichte (berechnet) | 1,649 Mg/m3 |
| R1 ; wR2 | 0,057; 0,149 |
| Wellenlänge | 1,54178 Å |

| | |
|---|---|
| a,b,c = Kantenlänge der Elementarzelle α,β,Y = Winkel der Elementarzelle Z = Anzahl der Moleküle in der Elementarzelle | |

Neben der Röntgenpulverdiffraktometrie und der kristallographischen Analyse kann man auch die Differentialscanningkalorimetrie (DSC) zur Identifizierung der Form II heranziehen.

Form II zeigt ein Thermogramm mit charakteristischem Schmelzpeak im Bereich von 170 und 200 °C. Das Peakmaximum liegt typischerweise im Bereich von etwa 180 °C bis190 °C. Die hier angegebenen Schmelzpunkte beziehen sich auf mittels Differentialkalorimetrie (Differential Scanning Calorimetry: DSC, Tiegelmaterial Aluminium, Aufheizrate 5 K/min) ermittelte Werte.

Die Herstellung der erfindungsgemäßen Form II des Phenyluracils I gelingt durch kontrollierte Kristallisation aus einer Lösung des Phenyluracils I in einem organischen Lösungsmittel, das im Wesentlichen frei ist von Wasser.

Hierzu stellt man in einem ersten Schritt i) eine Lösung des Phenyluracils I in einem organischen Lösungsmittel, das im Wesentlichen frei ist von Wasser, bereit und bewirkt anschließend in einem zweiten Schritt eine kontrollierte Kristallisation des Phenyluracils I.

Im Wesentlichen frei von Wasser bedeutet in diesem Zusammenhang, dass die Konzentration an Wasser in der das Phenyluracil I enthaltenden Lösung 10 Gew.-%, häufig 5 Gew.-% und insbesondere 1 Gew.-%, bezogen auf die Gesamtmenge an Lösungsmittel, nicht überschreitet.

Der Begriff "kontrollierte Kristallisation" ist so zu verstehen, dass die Kristallisation über einen längeren Zeitpunkt erfolgt, der in der Regel wenigstens 1 h, häufig wenigstens 2 h und insbesondere wenigstens 3 h beträgt. Die Kristallisation kann auch über einen längeren Zeitraum bis hin zu mehreren Tagen, z. B. 1, 2 bis 3 Tagen erfolgen. Häufig wird die Kristallisationsdauer jedoch 15 h nicht überschreiten. Dementsprechend erfolgt die Kristallisation in der Regel über einen Zeitraum von 1 bis 24 h, häufig 2 h bis 15 h, insbesondere 3 bis 10 h.

Geeignete Lösungsmittel sind grundsätzlich solche organischen Lösungsmittel und Lösungsmittelgemische, in denen das Phenyluracil I bei erhöhter Temperatur hinreichend löslich ist, z. B. bei 50 °C eine Löslichkeit von wenigstens 100 g/L aufweist.

Bevorzugt werden weiterhin Lösungsmittel und Lösungsmittelgemische, deren Siedepunkt bei Normaldruck im Bereich von 50 bis 160 °C liegt.

Beispiele für geeignete Lösungsmittel sind insbesondere die im Folgenden genannten organischen Lösungsmittel, die im Folgenden auch als Lösungsmittel L1 bezeichnet werden:
- C₁-C₆-Alkanole wie Methanol, Ethanol, Propanol, n-Butanol, Isobutanol, tert.-Butanol, 1-Pentanol oder Hexanol,
- acyclische Ketone mit 3 bis 8 C-Atomen wie Aceton, Methylethylketon oder 3-Methylbutan-2-on (Isopropylmethylketon),
- cyclische Ketone mit 5 bis 8 C-Atomen wie Cyclohexanon oder Cycloheptanon,
- aromatische Kohlenwasserstoffe und Kohlenwasserstoffgemische sowie aromatische Chlorkohlenwasserstoffe, insbesondere Mono- und Di-C₁-C₃-alkylbenzole wie Toluol, Xylole, Chlorbenzol und Dichlorbenzole,
- Di-C₁-C₆-alkylether wie Diethylether, Diisopropylether und Methyl-tert.-butylether,
- 5- oder 6-gliedrige alicyclischen Ether wie Tetrahydrofuran (THF) oder Dioxan,
- Nitroalkane mit 1 bis 3 C-Atomen wie Nitromethan,
- Alkylnitrile mit 2 bis 6 C-Atomen wie Acetonitril, Propionitril, Isobutyronitril und Butyronitril,
- C₁-C₄-Alkylester aliphatischer C₁-C₄-Carbonsäuren, insbesondere C₁-C₄-Alkylester der Essigsäure wie Ethylacetat und Butylacetat,
- N,N-Dimethylamide aliphatischer C₁-C₄-Carbonsäuren wie Dimethylformamid und Dimethylacetamid, sowie
- Gemische der vorgenannten Lösungsmittel.

Bevorzugte organische Lösungsmittel L1 sind
- C₂-C₄-Alkanole wie Methanol, Ethanol, Propanol, n-Butanol, Isobutanol und tert.-Butanol,
- acyclische Ketone mit 3 bis 6 C-Atomen wie Aceton, Methylethylketon oder 3-Methylbutan-2-on (Isopropylmethylketon),
- Mono-C₁-C₃-alkylbenzole wie Toluol,
- Di-C₁-C₆-alkylether wie Diethylether, Diisopropylether und Methyl-tert.-butylether,
- C₁-C₄-Alkylester der Essigsäure wie Ethylacetat und Butylacetat,
- 5- oder 6-gliedrige alicyclischen Ether wie Tetrahydrofuran (THF), sowie
- Gemische der vorgenannten Lösungsmittel.

Besonders bevorzugte organische Lösungsmittel L1 sind Mono-C₁-C₃-alkylbenzole, speziell Toluol sowie Gemische von Mono-C₁-C₃-alkylbenzolen, speziell von Toluol mit Tetrahydrofuran. Bevorzugt sind auch Gemische der bevorzugten Lösungsmittel L1, insbesondere Gemische von Mono-C₁-C₃-alkylbenzolen, speziell Gemische von Toluol mit Methanol, wobei bereits geringe Mengen an Methanol (z. B. bis 20 Vol.-%, insbesondere bis 10 Vol.-%) zu einer Verbesserung der Reinheit des erhaltenen Kristallisats führen.

Geeignet sind grundsätzlich auch Mischungen der vorgenannten organischen Lösungsmittel L1 mit davon verschiedenen Lösungsmitteln L2, wobei das Lösungsmittel L1 typischerweise die Hauptmenge, insbesondere wenigstens 70 Gew.-% und speziell wenigstens 90 Gew.-% des zur Kristallisation eingesetzten Lösungsmittels ausmacht. Insbesondere ist das Lösungsmittel L1 alleiniges Lösungsmittel oder enthält weniger als 5 Gew.-%, bezogen auf die Gesamtlösungsmittelmenge eines von L1 verschiedenen organischen Lösungsmittels.

Insbesondere handelt es sich bei den weiteren organischen Lösungsmitteln L2 um
- Carbonate mit vorzugsweise 2 bis 6 C-Atomen wie Dimethylcarbonat, Diethylcarbonat oder Ethylencarbonat,
- C₁-C₆-Alkylester aliphatischer C₁-C₄-Carbonsäuren wie Methylacetat, Ethylacetat, Propylacetat, Methylisobutyrat und Isobutylacetat,
- Hydroxy-C₁-C₄-alkylaromaten und C₁-C₄-Alkylcarbonalyaromaten wie Benzylalkohol und Acetophenon,
- aliphatische Chlorkohlenwasserstoffe wie Dichlormethan und Dichlorethan,
- Sulfoxide mit vorzugsweise 2 bis 6 C-Atomen wie Dimethylsulfoxid,
- Sulfone mit vorzugsweise 2 bis 6 C-Atomen wie Dimethylsulfon und Tetramethylensulfon, und
- Aliphatische und cycloaliphatische Kohlenwasserstoffe mit in der Regel 5 bis 10 C-Atomen wie Hexan, Cyclohexan, Petrolether und Petroleumbenzin.

Die Konzentration an Phenyluracil I in der zur Kristallisation eingesetzten Lösung hängt naturgemäß von der Art des Lösungsmittels und der Lösungstemperatur ab und liegt häufig im Bereich von 50 bis 800 g/L. Geeignete Bedingungen können vom Fachmann durch Routineexperimente ermittelt werden.

Vorzugsweise enthält die zur Kristallisation eingesetzte Lösung des Phenyluracils I, das Phenyluracil I in einer Reinheit von wenigstens 85 %, häufig wenigstens 90 %, insbesondere wenigstens 95 %, d. h. der Anteil organischer Verunreinigungen, die kein organisches Lösungsmittel sind, macht nicht mehr als 15 Gew.-%, häufig nicht mehr als 10 Gew.-% und insbesondere nicht mehr als 5 Gew.-%, bezogen auf das in dem Lösungsmittel gelöst vorliegende Phenyluracil I aus.

Die Lösung enthaltend das Phenyluracil I kann z. B. durch folgende Methoden bereitgestellt werden:
(1) Lösen des Phenyluracils I, vorzugsweise in einer von Form II verschiedenen Form, in einem organischen Lösungsmittel, das im Wesentlichen frei ist von Wasser; oder
(2) Herstellung des Phenyluracils I durch eine chemische Umsetzung und Überführung des Reaktionsgemischs, gegebenenfalls nach Abtrennen von Reagenzien und/oder Nebenprodukten, in ein erfindungsgemäß geeignetes organisches Lösungsmittel, das im Wesentlichen frei ist von Wasser.

Zur Herstellung der Lösung durch Lösen des Phenyluracils I kann grundsätzlich jede bekannte Form des Phenyluracils I eingesetzt werden. Naturgemäß wird man eine Form des Phenyluracils I wählen, die von der Form II verschieden ist. Hierzu kommen insbesondere eine feste oder flüssige Schmelze des Phenyluracils oder amorphes Phenyluracil I, wie aus dem Stand der Technik bekannt, in Betracht. Geeignete von Form I verschiedene Formen des Phenyluracils sind auch Solvate, insbesondere Hydrate des Phenyluracils I oder ein Methanol-Solvat des Phenyluracils I. Geeignet sind auch Mischungen unterschiedlicher Formen des Phenyluracils. Die Hydrate des Phenyluracils I sind Gegenstand einer parallelen Patentanmeldung auf die hier in vollem Umfang Bezug genommen wird.

Bei dem zum Lösen des Phenyluracils I verwendeten Lösungsmittel handelt es sich typischerweise um eines der vorgenannten organischen Lösungsmittel L1 oder um eine Mischung verschiedener Lösungsmittel L1 oder um ein Lösungsmittelgemisch, das wenigstens 70 Gew.-% und speziell wenigstens 90 Gew.-%, bezogen auf die Gesamtmenge des zum Lösen eingesetzten Lösungsmittels, an Lösungsmittel L1 enthält.

Zum Lösen der von Form II verschiedenen Form des Phenyluracils I wird man üblicherweise das Phenyluracil I als feinteiligen Feststoff oder als Schmelze unter Durchmischen in das Lösungsmittel bei einer Temperatur einarbeiten, bei der das Lösungsmittel bzw. Lösungsmittelgemisch das Phenyluracil I vollständig zu Lösen vermag.

Das Lösen der amorphen Form I folgt üblicherweise bei Temperaturen im Bereich von 20 bis 160 °C In einer bevorzugten Ausführungsform der Erfindung erfolgt das Lösen des Phenyluracils I bei erhöhter Temperatur, insbesondere bei wenigstens 50 °C, speziell bei wenigstens 80 °C, wobei die zum Lösen angewendete Temperatur naturgemäß den Siedepunkt des Lösungsmittels nicht überschreiten wird. Häufig erfolgt das Lösen bei Temperaturen im Bereich von 50 bis 140 °C, insbesondere im Bereich von 80 bis 120 °C und besonders bevorzugt im Bereich von 95 bis 115 °C.

Die Menge an Phenyluracil I, die im Lösungsmittel gelöst wird, hängt naturgemäß von der Art des Lösungsmittels L1 und der Lösungstemperatur ab und liegt häufig im Bereich von 100 bis 800 g/L. Geeignete Bedingungen können vom Fachmann durch Routineexperimente ermittelt werden.

Die Lösung des Phenyluracils I kann auch dadurch bereitgestellt werden, dass man ein durch eine chemische Umsetzung erhaltenes Reaktionsgemisch, welches das Phenyluracil I enthält, gegebenenfalls nach Abtrennen von Reagenzien und/oder Nebenprodukten, in ein erfindungsgemäß geeignetes organisches Lösungsmittel, das im Wesentlichen frei ist von Wasser, überführt. Dabei kann man so vorgehen, dass man die Umsetzung in einem organischen Lösungsmittel oder Lösungsmittelgemisch durchführt, das zumindest teilweise, vorzugsweise zu wenigstens 50 Gew.-% aus einem zur Kristallisation geeigneten Lösungsmittel besteht und, sofern erforderlich, eine Aufarbeitung durchführt, wobei man überschüssige Reagenzien und ggf. vorhandene Katalysatoren, ggf. vorhandenes, nicht geeignetes Lösungsmittel, z. B. Wasser und/oder Methanol entfernt. Die Herstellung einer Lösung des Phenyluracils I durch chemische Umsetzung einer geeigneten Vorstufe des Phenyluracils I, kann in Analogie zu den Methoden, die im eingangs zitierten Stand der Technik beschrieben sind, erfolgen, worauf hier in vollem Umfang Bezug genommen wird.

Sofern Solvate des Phenyluracils I zur Herstellung der Lösung eingesetzt werden, kann es vorteilhaft sein, das Solvat-Lösungsmittel nach dem Lösen jedoch vor der Kristallisation zu entfernen, beispielsweise durch Destillation.

Die Kristallisation der Form II des Phenyluracils I kann in üblicher Weise bewirkt werden, z. B.
- durch Abkühlen der Lösung, welche das Phenyluracil I gelöst enthält,
- durch Zugabe eines die Löslichkeit vermindernden organischen Lösungsmittels zu der Lösung, welche das Phenyluracil I gelöst enthält, insbesondere durch Zugabe eines wasserfreien unpolaren organischen Lösungsmittels;
- durch Einengen der Lösung, welche das Phenyluracil I gelöst enthält, oder
- durch eine Kombination der vorgenannten Maßnahmen

Die Kristallisation wird in der Regel soweit geführt, dass wenigstens 80 Gew.-%, vorzugsweise wenigstens 90 Gew.-%, des eingesetzten Phenyluracils I auskristallisieren.

In einer bevorzugten Ausführungsform der Erfindung wird man so vorgehen, dass man die Lösung, welche das Phenyluracil I gelöst enthält, bei erhöhter Temperatur, vorzugsweise bei wenigstens 50 °C, z. B. 50 bis 150 °C, vorzugsweise 80 bis 120 °C und besonders bevorzugt im Bereich von 100 bis 115 °C herstellt, und anschließend die Kristallisation des Phenyluracils I durch Abkühlen und gegebenenfalls Einengen der Lösung bewirkt. Vorzugsweise kühlt man die Lösung des Phenyluracils I um wenigstens 20 K, insbesondere um 30 bis 60 K ab, um die Kristallisation einzuleiten. Der Vorgang des Abkühlens kann in kontrollierter Weise durchgeführt werden, d. h. man kühlt mit einer geringen Abkühlrate von in der Regel nicht mehr als 20 K/h, z. B. 0,5 bis 20 K/h und häufig 1 bis 15 K/h ab. Vorteilhafterweise wird das kontrollierte Abkühlen mit Beginn der Kristallisation durchgeführt. Man kann aber auch rascher abkühlen und man wird dann das Kristallisat über einen längeren Zeitraum in der Mutterlauge bewegen, d. h. bis die gewünschte Kristallisationsdauer erreicht ist, bevor man es isoliert.

Zur Verbesserung der Reinheit kann die Kristallisation so durchgeführt werden, dass man zunächst die Lösung Phenyluracils herunterkühlt, bis ein Teil oder die Gesamtmenge des Phenyluracils auskristallisiert ist, anschließend wieder erhitzt, um das Kristallisat anzulösen, jedoch ohne dass ein vollständiges Lösen des Kristallisates eintritt, und abschließend wieder abkühlt. Bezüglich der Temperaturen und Abkühlraten gilt das zuvor Gesagte analog.

Die Kristallisation der Form II lässt sich durch Animpfen mit Impfkristallen der Form II fördern oder beschleunigen, beispielsweise indem vor oder während der Kristallisation Impfkristalle der Form II zugesetzt werden.

Sofern man bei der Kristallisation Impfkristalle zusetzt, beträgt ihre Menge typischerweise 0,001 bis 10 Gew.-%, häufig 0,005 bis 5 Gew.-%, insbesondere 0,01 bis 1 Gew.-% und speziell 0,05 bis 0,5 Gew.-%, bezogen auf das gelöste Phenyluracil I.

Sofern man die Kristallisation in Gegenwart von Impfkristallen der Form II durchführt, werden diese vorzugsweise erst bei einer Temperatur zugegeben, bei der die Sättigungskonzentration des Phenyluracils I in dem jeweiligen Lösungsmittel erreicht ist, d. h. bei oder unterhalb derjenigen Temperatur, bei der die gelöste Menge an Phenyluracil I in dem jeweiligen Lösungsmittel eine gesättigte Lösung bildet. Die Temperaturabhängigkeit der Sättigungskonzentration in einem Lösungsmittel kann der Fachmann in Routineexperimenten ermitteln. Häufig erfolgt die Zugabe der Impfkristalle, wenn die Temperatur der Lösung nicht mehr als 60 °C beträgt. Vorzugsweise lässt man nach der Zugabe der Impfkristalle die Lösung auf Temperaturen unterhalb 30 °C, insbesondere von 25 °C oder darunter, z. B. auf Temperaturen im Bereich von 0 °C bis 25 °C abkühlen, bevor man zur Isolierung der Form II des Phenyluracils I das erhaltene kristalline Material von der Mutterlauge abtrennt. Das Abkühlen bei Anwesenheit von Impfkristallen kann kontrolliert mit einer Abkühlrate von in der Regel nicht mehr als 30 K/h, z. B. 1 bis 30 K/h, häufig 2 bis 20 K/h und insbesondere 3 bis 15 K/h oder nicht kontrolliert erfolgen.

Auch hier kann ein Wiederaufheizen zum Anlösen des Kristallisates mit anschließender erneuter Abkühlung, wie zuvor beschrieben, zur Verbesserung der Produktreinheit führen.

Es hat sich bewährt, wenn man das kristalline Material noch eine Zeit lang bei Temperaturen unterhalb der Kristallisationstemperatur, z. B. im Bereich von 0 bis 35 °C in der Mutterlauge bewegt, z. B. 1 h bis 3 h, um eine vollständige Kristallisation in die Form II zu gewährleisten. Die Gesamtdauer vom Beginn des Abkühlens bis zur Isolierung der Kristalle durch Abtrennung der Mutterlauge liegt dann in den oben genannten Bereichen.

Alternativ kann man die Kristallisation auch durch Zugabe eines wasserfreien unpolaren Lösungsmittels L2, z. B. von 5 bis 60 Vol.-%, insbesondere 20 bis 55 Vol.-% und speziell von 30 bis 50 Vol.-%, bezogen auf das Volumen des zum Lösen des Phenyluracils 1 verwendeten Lösungsmittels bzw. Lösungsmittelgemischs bewirken. Vorzugsweise erfolgt die Zugabe des unpolaren Lösungsmittels L2 über einen längeren Zeitraum, z. B. über einen Zeitraum von 30 min bis 10 h, insbesondere über einen Zeitraum von 1 h bis 8 h.

Insbesondere kann man die Zugabe des unpolaren Lösungsmittels und den Zusatz von Impfkristallen miteinander kombinieren. Die Zugabe des unpolaren Lösungsmittels kann in Form von reinem unpolaren Lösungsmittel oder in Form eines Gemisches von unpolarem Lösungsmittel mit einem der vorgenannten Lösungsmittel L1, insbesondere im Gemisch mit dem zum Lösen eingesetzten Lösungsmittel, erfolgen. Beispiele für unpolare Lösungsmittel sind aliphatische und cycloaliphatische Kohlenwasserstoffe wie Pentan Hexan, Cyclohexan, Isohexan, Heptan, Octan, Decan, sowie Halogenaromaten wie Chlorbenzol, Dichlorbenzol oder Gemische davon.

Die Gewinnung der Form II aus dem Kristallisat, d. h. die Abtrennung der Form II von der Mutterlauge, gelingt durch übliche Techniken zur Trennung fester Bestandteile von Flüssigkeiten, z. B. durch Filtration, Zentrifugieren oder durch Dekantieren. In der Regel wird man den isolierten Feststoff waschen, z. B. mit dem zur Kristallisation verwendeten Lösungsmittel, mit Wasser oder mit einem Gemisch aus dem zur Kristallisation verwendeten organischen Lösungsmittel mit Wasser. Das Waschen kann in ein oder mehreren Schritten erfolgen, wobei man häufig im letzten Waschschritt mit Wasser wäscht. Das Waschen erfolgt typischerweise bei Temperaturen unterhalb 30 °C, häufig unterhalb 25 °C und insbesondere unterhalb 20 °C, um den Verlust an Wertprodukt möglichst gering zu halten. Anschließend kann man die erhaltene Form II trocknen und dann der Weiterverarbeitung zuführen. Häufig wird man jedoch den nach Waschen erhaltenen feuchten Wirkstoff, insbesondere einen wasserfeuchten Wirkstoff direkt der weiteren Verarbeitung zuführen.

Durch die erfindungsgemäße Kristallisation wird die Form II mit einem Gehalt an Phenyluracil I von wenigstens 94 Gew.-%, insbesondere wenigstens 96 Gew.-% erhalten. Der Anteil an Form II, bezogen auf die Gesamtmenge an Phenyluracil I liegt typischerweise bei wenigstens 90 %, häufig wenigstens 95 % und insbesondere wenigstens 98%.

Die Herstellung des als Ausgangsmaterial für die Herstellung der Form II verwendeten 2-Chlor-5-[3,6-dihydro-2,6-dioxo-4-(trifluormethyl)-1 (2H)-pyrimidinyl]-4-fluor-N-[[methyl(1-methylethyl)-amino]sulfonyl]-benzamid kann nach den in WO 01/083459, WO 03/097589, WO 05/054208, WO 06/097589 und PCT/EP 2006/062414 beschriebenen Verfahren erfolgen, auf die hiermit in vollem Umfang Bezug genommen wird.

Besonders bevorzugt wird das Phenyluracil I nach folgenden Verfahren hergestellt:
1) Umwandlung der 2-Chlor-5-[3,6-dihydro-2,6-dioxo-4-(trifluormethyl)-1 (2H)-pyrimidinyl]-4-fluor-benzoesäure in ihr Säurechlorid oder das entsprechende Anhydrid und anschließende Umsetzung des entsprechenden aktivierten Säurederivats mit N-Methyl-N-(1-methylethyl)sulfamoylamid, z. B.: 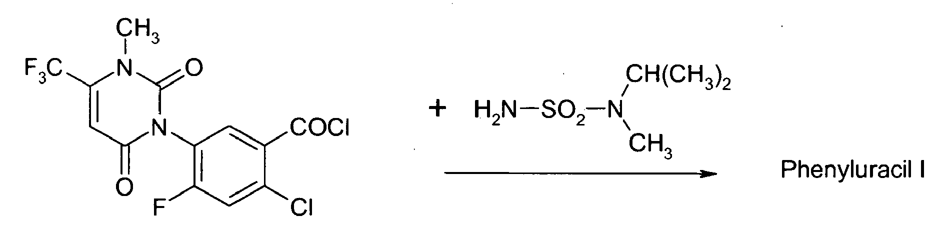
   Diese Umsetzung erfolgt üblicherweise bei Temperaturen von 20 °C bis zum Siedepunkt der Reaktionsmischung in einem organischen Lösungsmittel in Gegenwart einer Base sowie gegebenenfalls eines Katalysators [vgl. z. B. WO 01/083459, WO 03/097589 und auch WO 04/039768].
   Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid. Es können auch Gemische der genannten Lösungsmittel verwendet werden.
   Als Basen kommen allgemein anorganische Basen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z. B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht.
   Die Basen werden im Allgemeinen in katalytischen oder äquimolaren Mengen eingesetzt, sie können aber auch im Überschuss oder gegebenenfalls als Lösungsmittel verwendet werden.
   Die Edukte werden im Allgemeinen in äquimolaren Mengen miteinander umgesetzt. Es kann vorteilhaft sein, eines der Edukte in einem Überschuss einzusetzen.
2) Methylierung von 2-Chlor-5-[3,6-dihydro-2,6-dioxo-4-(trifluormethyl)-1 (2H)-pyrimidinyl]-4-fluor-N-[[methyl(1-methylethyl)amino]sulfonyl]-benzamid (im Folgenden "NH-Uracil") mit einem Methylierungsmittel C:

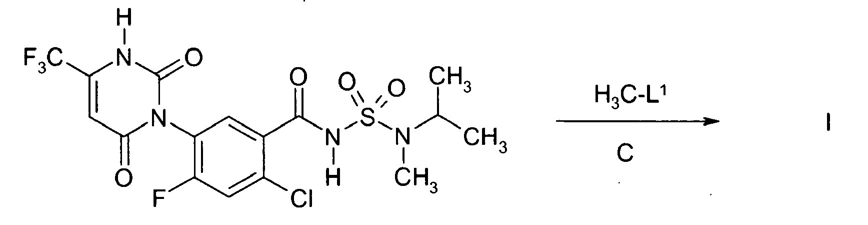

Die Gruppe L¹ steht für eine nucleophile Abgangsgruppe, bevorzugt für Halogen wie Chlor, Brom oder lod, C₁-C₆-Alkylsulfat wie Methylsulfat, C₁-C₆-Alkylsulfonyloxy wie Methylsulfonyloxy, C₁-C₆-Halogenalkylsulfonyloxy wie Trifluormethylsulfonyloxy oder Phenylsulfonyloxy; sehr bevorzugt für C₁-C₆-Alkylsulfat.

Geeignete Methylierungsmittel C sind Methylhalogenide wie Methyliodid, Methylbromid, Methylchlorid, Dimethylsulfat, C₁-C₆-Halogenalkylsulfonsäure-methylester oder Phenylsulfonsäuremethylester, besonders bevorzugt sind Methylhalogenide und Dimethylsulfat; außerordentlich bevorzugt ist Dimethylsulfat.

Das Methylierungsmittel C kann sowohl in äquimolarer Menge, bezogen auf das NH-Uracil, als auch in substöchiometrischer Menge oder überstöchiometrischer Menge eingesetzt werden.

Üblicherweise wird das Verfahren (2) in Gegenwart einer Base durchgeführt, wobei alle üblichen organischen und anorganischen Basen, z. B. die Verfahren (1) genannten Basen, in Betracht kommen. Bevorzugt sind Basen ausgewählt unter Alkali- und Erdalkalimetallhydroxiden wie Lithiumhydroxid, Natriumhydroxid, Kaliumhydroxid und Calciumhydroxid, Alkali- und Erdalkalimetalloxiden wie Lithiumoxid, Natriumoxid, Calciumoxid und Magnesiumoxid, Alkali- und Erdalkalimetallcarbonaten wie Lithiumcarbonat, Natriumcarbonat, Kaliumcarbonat und Calciumcarbonat sowie Alkalimetallhydrogencarbonaten wie Natriumhydrogencarbonat. In einer besonders bevorzugten Ausführungsform setzt man als Base Natrium- oder Kaliumhydroxid ein. Die Basen werden im Allgemeinen in äquimolaren Mengen bezogen auf das NH-Uracil eingesetzt, sie können aber auch katalytisch, im Überschuss oder gegebenenfalls als Lösungsmittel verwendet werden.

In einer sehr bevorzugten Variante des Verfahrens (2) wird der pH-Wert während der gesamten Umsetzung durch kontinuierliche oder portionsweise Basenzugabe in einem Bereich von 1 bis 6 gehalten. "Portionsweise Basenzugabe" bedeutet, dass die Basenzugabe während der Umsetzung in einzelnen Portionen erfolgt, d. h. in mindestens 2 Portionen, oder in mehreren bis zu vielen Portionen, oder kontinuierlich.

Zur Umsetzung können das NH-Uracil, das Methylierungsmittel C und gegebenenfalls die Base getrennt, gleichzeitig oder nacheinander in das Reaktionsgefäß eingebracht und zur Reaktion geführt werden.

Gemäß einer ersten Ausführungsform des Verfahrens (2) erfolgt die Umsetzung des NH-Uracils mit dem Methylierungsmittel C in einem organischen Lösungsmittel.

Als Lösungsmittel für diese Umsetzungen kommen, je nach Temperaturbereich, aliphatische, cycloaliphatische oder aromatische Kohlenwasserstoffe wie Pentan, Hexan, Cyclopentan, Cyclohexan, Toluol, Xylol, chlorierte aliphatische und aromatische Kohlenwasserstoffe wie Dichlormethan, Trichlormethan, 1,2-Dichlorethan, 1,1,2,2-Tetrachlorethan, Chlorbenzol, 1,2-, 1,3- oder 1,4-Dichlorbenzol, Chlortoluole, Dichlortoluole, offfenkettige Dialkylether wie Diethylether, Di-n-proplyether, Di-n-isopropylether, Methyl-tert-butylether, cyclische Ether wie Tetrahydrofuran, 1,4-Dioxan, Anisol, Glykolether wie Dimethylglykolether, Diethylglykolether, Diethylenglykoldimethylether, Diethylenglykoldiethylether, C₁-C₄-Alkohole wie Methanol, Ethanol, n-Propanol, iso-Propanol, n-Butanol, aliphatische Carbonsäure-C₁-C₆-alkylester wie Methylacetat, Ethylacetat oder n-Butylacetat; Ketone wie Aceton, Methylethylketon, Methylisopropylketon, Methylisobutylketon, Butanon, Carbonate wie Diethylcarbonat und Ethylencarbonat, N,N-Dialkylamide wie N,N-Dimethylformamid oder N,N-Dimethylacetamid, N-Alkyllactame wie N-Methylpyrrolidon, Sulfoxide wie Dimethylsulfoxid, Tetraalkylharnstoffe wie Tetramethylharnstoff, Tetraethylharnstoff, Tetrabutylharnstoffe, Dimethylethylenharnstoff, Dimethylpropylenharnstoff oder Gemische dieser Lösungsmittel in Betracht.

Als Lösungsmittel bevorzugt sind N,N-Dialkylamide wie N,N-Dimethylformamid, N-Alkyllactame wie N-Methylpyrrolidon, Ketone wie Aceton, aromatische Kohlenwasserstoffe wie Toluol, chlorierte aliphatische und aromatische Kohlenwasserstoffe wie Dichlormethan oder Chlorbenzol, *cyclische Ether wie Tetrahydrofuran, aliphatische Carbonsäure-C₁-C₆-alkylester wie Ethylacetat, Butylacetat oder Gemische dieser Lösungsmittel.

Vorzugsweise führt man die Methylierung des NH-Uracils bei Temperaturen zwischen -5 °C und 100 °C durch. Die Reaktionszeit kann der Fachmann in an sich üblicher Weise durch Routinemethoden wie Dünnschichtchromatographie oder HPLC ermitteln.

In einer anderen Variante des Verfahrens (2a) kann die Umsetzung auch in einem Mehrphasensystem durchgeführt werden. Diese Variante ist bevorzugt.

Bezüglich Methylierungsmittel C, pH-Wert, Base, Temperatur und Druck gilt das zuvor Gesagte.

Gemäß einer zweiten, bevorzugten Ausführungsform des Verfahrens (2) erfolgt die Umsetzung des NH-Uracils mit dem Methylierungsmittel C in einem wässrig-organischen Mehrphasensystem in Gegenwart eines oder mehrerer Phasentransferkatalysatoren.

Beispiele für Phasentransferkatalysatoren sind quartäre Ammoniumsalze, Phosphoniumsalze, Kronenether oder Polyglycole. Bevorzugte geeignete quartäre Ammoniumsalze umfassen z. B. Tetra-(C₁-C₁₈)-alkylammonium-halogenide und N-Benzyltri-(C₁-C₁₈)-alkylammonium-halogenide. Bevorzugte geeignete Phosphoniumsalze umfassen z. B. C₁-C₁₈-Alkyltriphenylphosphoniumchloride, -bromide, -acetate, Tetra-(C₁-C₁₈)-alkylphosphoniumchloride oder -bromide Tetraphenylphosphoniumchlorid oder -bromid, Benzyltriphenylphosphonium chlorid oder-bromid. Bevorzugte geeignete Kronenether umfassen z. B. 18-Krone-6, Dibenzo-18-Krone-6. Bevorzugte geeignete Polyglycole umfassen z. B. Diethylenglycol-dibutylether (=Butyldiglyme), Tetraethylenglycol-dimethylether (= Tetraglyme), Triethylenglycol-dimethylether (= Triglyme), Polyglycol-dimethylether. In der Regel setzt man den Phasentransferkatalysator in einer Menge von bis zu 20 mol-%, bezogen auf das NH-Uracil ein.

Das Mehrphasensystem umfasst eine wässrige Phase und wenigstens eine organische flüssige Phase. Daneben können auch feste Phasen auftreten.

Die wässrige Phase ist vorzugsweise eine Lösung, welche die Base enthält, insbesondere eine wässrige Lösung von Alkali- oder Erdalkalihydroxiden (wie Lithiumhydroxid, Natriumhydroxid, Kaliumhydroxid und Calciumhydroxid), -carbonaten (wie Lithiumcarbonat, Natriumcarbonat, Kaliumcarbonat und Calciumcarbonat) oder Alkalimetallhydrogencarbonaten (wie Natriumhydrogencarbonat) in Wasser. Besonders bevorzugt verwendet man Alkali- oder Erdalkalihydroxide, sehr bevorzugt Natriumhydroxid.

Die Base(n) wird (werden) im Allgemeinen in äquimolaren Mengen bezogen auf das NH-Uracil eingesetzt, sie können aber auch katalytisch, im Überschuss oder gegebenenfalls als Lösungsmittel verwendet werden. Vorzugsweise setzt man wenigstens eine äquimolare Menge an Base, bezogen auf das NH-Uracil ein.

Für die organische Phase kommen vorzugsweise als Lösungsmittel je nach Temperaturbereich aliphatische, cycloaliphatische oder aromatische Kohlenwasserstoffe wie Pentan, Hexan, Cyclopentan, Cyclohexan, Toluol, Xylol, chlorierte aliphatische und aromatische Kohlenwasserstoffe wie Dichlormethan, Trichlormethan, 1,2-Dichlorethan, 1,1,2,2-Tetrachlorethan, Chlorbenzol, 1,2-, 1,3- oder 1,4-Dichlorbenzol, Chlortoluole, Dichlortoluole, offenkettige Dialkylether wie Diethylether, Di-n-proplyether, Di-n-isopropylether, Methyl-tert-butylether, cyclische Ether wie Tetrahydrofuran (THF) und Anisol, aliphatische Carbonsäure-C₁-C₆-alkylester wie Methylacetat, Ethylacetat oder n-Butylacetat oder Gemische dieser Lösungsmittel in Betracht. Für die organische Phase sind Ethylacetat, n-Butylacetat, Chlorbenzol, THF, Toluol oder Gemische dieser Lösungsmittel als Lösungsmittel bevorzugt; sehr bevorzugt sind Ethylacetat, n-Butylacetat, Chlorbenzol und THF-Gemische sowie Toluol und THF-Gemische.

Während der Umsetzung können feste Phasen auftreten, wenn z. B. das NH-Uracil, das Methylierungsmittel C, die Base und/oder der Phasentransferkatalysator nicht vollständig gelöst sind.

In einer bevorzugten Ausführungsform besteht das Mehrphasensystem als wässrige Phase aus wässriger Natriumhydroxid-Lösung, und als organische Phase aus Toluol und Tetrahydrofuran, oder Dichlormethan und Tetrahydrofuran, Chlorbenzol und Tetrahydrofuran, oder aus Ethylacetat oder n-Butylacetat.

Zur Umsetzung können das NH-Uracil, die Methylierungsmittel C, die Base und der gegebenenfalls Phasentransferkatalysator getrennt, gleichzeitig oder nacheinander in das Reaktionsgefäß eingebracht und zur Reaktion geführt werden.

In der Regel wird man bei Verwendung eines Zweiphasensystems vor der Kristallisation der Form II die Phasen trennen. Besonders bevorzugt trocknet man das auf diesem Weg erhaltene Produkt vor der Kristallisation durch dem Fachmann bekannte Trocknungsmethoden, beispielsweise durch azeotropes Abdestillieren des Wasser zusammen mit einem Teil des organischen Lösungsmittels..

Die folgenden Abbildungen und Beispiele dienen der Erläuterung der Erfindung und sind nicht einschränkend zu verstehen.

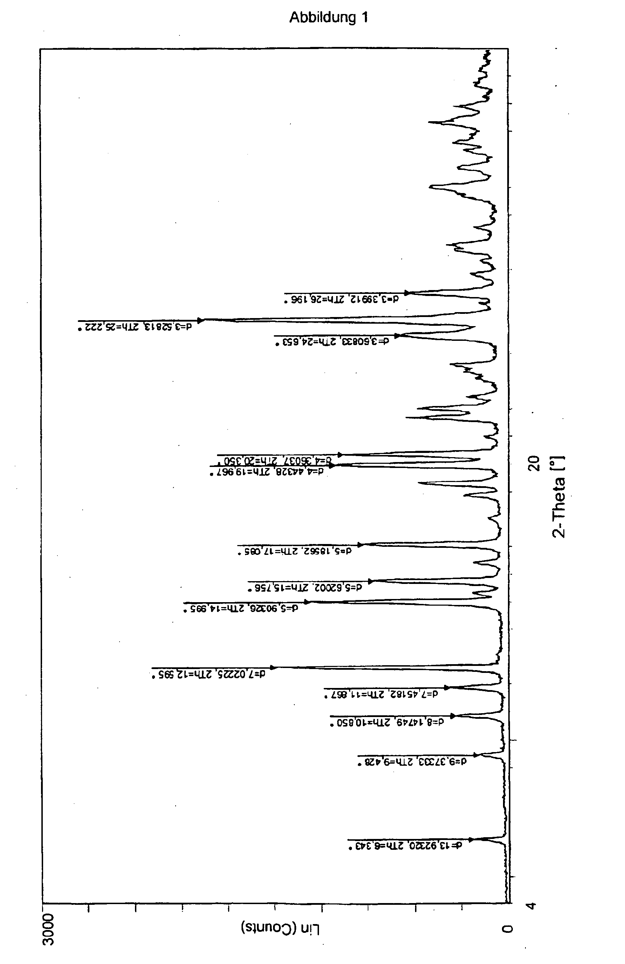

Abbildung 1 zeigt ein Röntgenpulverdifraktogramm der Form II. Das Röntgendiffraktogramm der Form II wurde mit einem Diffraktometer D-5000 der Fa. Bruker-AXS in Reflexionsgeometrie im Bereich von 2θ = 4° - 35° mit einer Schrittweite von 0,02° unter Verwendung der Cu-K_{α}-Strahlung bei 25 °C aufgenommen. Aus ermittelten 2θ-Werten wurden die angegebenen Netzebenenabstände d berechnet.

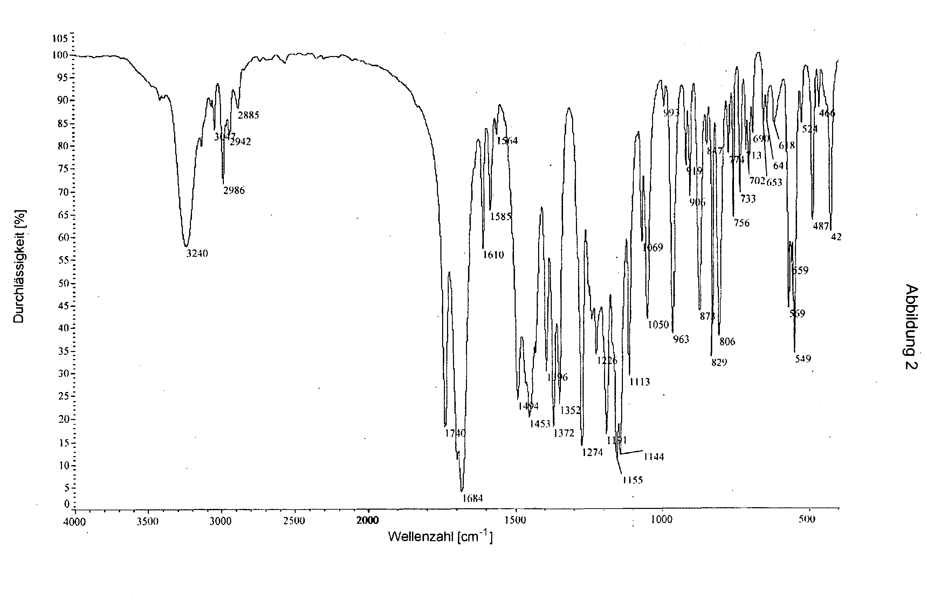

Abbildung 2 zeigt ein IR-Spektrum der Form II. Die IR-Spektren wurden mittels FTIR-Spectrometern "Nicolet Magna 550" und "Nicolet Magna 750" der Fa. Thermo Electron Corp./USA im Wellenzahlenbereich von 400 - 4000 cm⁻¹ bei einer Auflösung von 4 cm⁻¹ (32 Scans) aufgenommen. Als Probenkörper dienten KBr-Presslinge.

Die Bestimmung der Schmelzpunkte und Schmelzwärmen erfolgte mittels DSC mit einem Mettler Toledo DSC 25 der Fa. Mettler mit einer Aufheizrate von 5 K/min im Bereich von -5° bis +80 °C. Die Probenmenge betrug 5 bis 10 mg.

Die Bestimmung der kristallographischen Daten der Form II (Tabelle 1) erfolgte an einem Einkristalldiffraktometer der Fa. Bruker ("Bruker P4") unter Verwendung von Cu-K_{α}-Strahlung.

### Herstellung der Form II des Phenyluracils I durch Kristallisation der amorphen Form I aus einem organischen Lösungsmittel unter Entfernen des Lösungsmittels (allgemeine Vorschrift)

1 g amorphes Phenyluracil I wurden in 25 ml des jeweils angegebenen Lösungsmittels bei Raumtemperatur gelöst. Die erhaltene Lösung wurde auf die in Tabelle 3 angegebene Temperatur erwärmt und bei dieser Temperatur belassen, wobei man zum Verdampfen des Lösungsmittels einen Stickstoffstrom über die Lösung leitete. Nachdem das Lösungsmittel entfernt war, kühlte man auf Umgebungstemperatur und isolierte das kristalline Material und analysierte mittels DSC und/oder mittels Röntgenpulverdiffraktometrie (XRD). In allen Fällen wurde die Form II erhalten.

**Tabelle 3**

| Beispiel | Lösungsmittel | T [°C] | XRD¹⁾ | DSC-Peak [°C] |
|---|---|---|---|---|
| 1 | Aceton | 35 | n.a. | 187 |
| 2 | Isopropanol | 35 | + | 183, 187 |
| 3 | Isopropanol | 70 | n.a. | 190 |
| 4 | Toluol | 35 | + | 189 |
| 5 | Toluol | 80 | + | 189 |
| 6 | Methylisobutylketon | 100 | n.a. | 189 |
| 7 | 1-Pentanol | 50 | n.a. | 188 |
| 8 | 1-Pentanol | 120 | + | 178 |
| 9 | Nitromethan | 40 | + | 186 |

| | | | | |
|---|---|---|---|---|
| 1) Röntgenpulverdiffraktogramm: + = gemessen; n.a. nicht gemessen | | | | |

Beispiel 10: 1 g amorphes Phenyluracil I wurden bei Raumtemperatur in 25 ml Acetonitril gelöst. Die klare Lösung ließ man eine Woche offen bei Umgebungstemperatur stehen, wobei das Lösungsmittel weitgehend verdampfte und ein kristalliner weißer Niederschlag zurückblieb. Der DSC-Peak bei 187 °C und bestätigte das Vorliegen der Form II.

### Beispiel 11: Herstellung der Form II des Phenyluracils I durch Kristallisation der amorphen Form I aus Aceton

Man löste 0,2 g der amorphen Form I in 10 Tropfen Aceton unter Rühren bei 22 °C. Danach rührte man weitere 3 Minuten und es bildete sich eine erste Trübung, die sich während der nächsten 30 Minuten unter Bildung eines Niederschlages verstärkte. Man ließ den Niederschlag absitzen (30 min.) und entfernte dann das Aceton im Vakuum und erhielt auf diese Weise 0,191 g (96 % der Theorie) der Form II mit einem Schmelzpunkt von 180 - 184 °C.

### Beispiel 12: Herstellung der Form II des Phenyluracils I durch Kristallisation aus der Reaktionslösung

50,0 g (0,098 mol) 2-Chlor-5-[3,6-dihydro-2,6-dioxo-4-(trifluormethyl)-1 (2H)-pyrimidinyl]-4-fluor-N-{[methyl-(1-methylethyl)amino]sulfonyl}-benzamid, 3,2 g (0,0089 mol) Tetrabutylammoniumbromid (= TBAB) und 15,1 g (0,12 mol) Dimethylsulfat wurden bei 25 °C in einem Gemisch aus Toluol, Wasser und THF vorgelegt und der Ansatz auf 40 °C aufgeheizt. Anschließend stellte man durch Zugabe von wässriger 10%iger NaOH-Lösung im Reaktionsgemisch einen pH-Wert von 5,3 - 5,5 ein. Während der ganzen Reaktionsdauer wurde weiterhin wässrige 10%ige NaOH-Lösung zugegeben, so dass der pH-Wert über den gesamten Reaktionsverlauf konstant bei dem zuvor eingestellten pH-Wert Iag. Nach beendeter Reaktion wurde das Reaktionsgemisch noch 3,5 h bei 40 °C nachgerührt. Anschließend wurden die Phasen getrennt.

Aus der so erhaltenen Lösung wurden 55 bis 60 % des eingesetzten Lösungsmittels durch Destillation bei Normaldruck entfernt, wobei man eine heiße Lösung der Titelverbindung in Toluol erhielt. Die Lösung wurde anschließend auf 70 °C und dann innerhalb von 5 h mit konstanter Abkühlrate auf 20 °C abgekühlt und 3 h bei 20 °C nachgerührt. Der ausgefallene Feststoff wurde abgesaugt und getrocknet. Man erhielt 42,6 g (84,0 %) der Titelverbindung als Form II mit einem Wirkstoffgehalt von 96,8 %.

Die Form II eignet sich ebenso wie die Form I als Herbizid, ist jedoch dieser bezüglich Wirksamkeit überlegen. Die Erfindung betrifft daher auch Pflanzenschutzmittel, enthaltend die kristalline Form II und für die Formulierung von Pflanzenschutzmitteln übliche Hilfsmittel, insbesondere Pflanzenschutzmittel in Form von wässrigen oder nicht-wässrigen Suspensionskonzentraten. Die Erfindung betrifft auch ein Verfahren zur Bekämpfung von unerwünschtem Pflanzenwuchs, das dadurch gekennzeichnet, dass man die Form II des Phenyluracils, vorzugsweise als geeignete Wirkstoffaufbereitung auf Pflanzen, deren Lebensraum und/oder auf Samen einwirken lässt.

Die die Form II enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode kann die Form II bzw. können die sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus armeniaca, Prunus avium, Prunus cerasus, Prunus dulcis, Prunus domesticua, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus kann die Form II bzw. können die sie enthaltenden herbiziden Mittel auch in Kulturen, die durch Züchtung einschließlich gentechnischer Methoden gegen die Wirkung von Herbiziden tolerant sind, verwendet werden.

Weiterhin kann die Form II bzw. können die sie enthaltenden herbiziden Mittel auch in Kulturen, die durch Züchtung einschließlich gentechnischer Methoden gegen Insekten- oder Pilzbefall tolerant sind, verwendet werden.

Des Weiteren wurde gefunden, dass die Form II auch zur Defoliation und Desikkation von Pflanzenteilen geeignet ist, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und /oder Defoliation von Pflanzen, Verfahren zur Herstellung dieser Mittel und Verfahren zur Desikkation und/oder Defoliation von Pflanzen mit der Form II gefunden.

Als Desikkantien eignet sich die Form II insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht. Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, d. h. die Förderung der Ausbildung von Trenngewebe zwischen Frucht- oder Blatt- und Sprossteil der Pflanzen ist auch für ein gut kontrollierbares Entblättern von Nutzpflanzen, insbesondere Baumwolle, wesentlich. Außerdem führt die Verkürzung des Zeitintervalls, in dem die einzelnen Baumwollpflanzen reif werden, zu einer erhöhten Faserqualität nach der Ernte.

Des Weiteren wurde gefunden, dass die Form II auch zur Kontrolle von Koniferen, insbesondere von wildwachsenden Koniferensämlingen, speziell zur Kontrolle von wildwachsenden Kiefernsämlingen geeignet ist.

Weiterhin eignet sich die Form II auch zur Kontrolle von Unkräutern in Kulturpflanzen, wie z. B. Sojabohne, Baumwolle, Raps, Flachs, Linsen, Reis, Zuckerrübe, Sonnenblume, Tabak und Getreide, wie z. B. Mais oder Weizen.

Die Form II bzw. die sie enthaltenden herbiziden Mittel können beispielsweise in Form von direkt versprühbaren wässrigen Lösungen, Pulvern, Suspensionen, auch hochprozentigen wässrigen, öligen oder sonstigen Suspensionen, Ölsuspensionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge der Form II und für die Formulierung von Pflanzenschutzmitteln übliche Hilfsmittel und Trägerstoffe.

Als Trägerstoffe kommen grundsätzlich alle festen Substanzen in Betracht, die üblicherweise in Pflanzenschutzmitteln, insbesondere in Herbiziden zum Einsatz kommen. Feste Trägerstoffe sind z. B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Im Falle von flüssigen Formulierungen der Form II weisen die Zusammensetzungen eine flüssige Phase auf. Als flüssige Phase kommen grundsätzlich Wasser sowie solche organischen Lösungsmittel in Betracht, in denen die Form II nur eine geringe oder keine Löslichkeit besitzt, z. B. solche, in denen die Löslichkeit der Form II des Phenyluracils I bei 25 °C und 1013 mbar nicht mehr als 1 Gew.-%, insbesondere nicht mehr als 0,1 Gew,-% und speziell nicht mehr als 0,01 Gew.-% beträgt.

Bevorzugte flüssige Phasen sind insbesondere Wasser und wässrige Lösungsmittel, d. h. Lösungsmittelgemische, die neben Wasser noch bis zu 30 Gew.-%, vorzugsweise jedoch nicht mehr als 10 Gew.-%, bezogen auf die Gesamtmenge an Wasser und Lösungsmittel, eines oder mehrere mit Wasser mischbare organische Lösungsmittel, z. B. mit Wasser mischbare Ether wie Tetrahydrofuran, Methylglykol, Methyldiglykol, Alkanole wie Methanol, Ethanol, Isopropanol oder Polyole wie Glykol, Glycerin, Diethylenglykol, Propylenglykol und dergleichen, enthalten.

Bevorzugte flüssige Phasen sind weiterhin nicht-wässrige organische Lösungsmittel, in denen die Löslichkeit der Form II des Phenyluracils I bei 25 °C und 1013 mbar nicht mehr als 1 Gew.-%, insbesondere nicht mehr als 0,1 Gew.-% und speziell nicht mehr als 0,01 Gew.-% beträgt. Hierzu zählen insbesondere aliphatische und cycloaliphatische Kohlenwasserstoffe sowie Öle, insbesondere solche pflanzlichen Ursprungs, weiterhin C₁-C₄-Alkylester von gesättigten oder ungesättigten Fettsäuren oder Fettsäuregemischen, insbesondere die Methylester, z. B. Ölsäuremethylester, Stearinsäuremethylester, Rapsölmethylester, aber auch paraffinische Mineralöle und dergleichen.

Typische Hilfsmittel umfassen oberflächenaktive Substanzen, insbesondere die in Pflanzenschutzmitteln üblicherweise eingesetzten Netzmittel und Dispergier(hilfs)mittel, weiterhin die Viskosität verändernde Additive (Verdicker, Andicker), Antischaummittel, Frostschutzmittel, Mittel zur Einstellung des pH-Wertes, Stabilisatoren, Anticaking-Mittel und Biozide (Konservierungsmittel).

Die Erfindung betrifft insbesondere Mittel für den Pflanzenschutz in Form eines wässrigen Suspensionskonzentrats (SC). Derartige Suspensionskonzentrate enthalten die Form II des Phenyluracils I in einer feinteiligen partikulären Form, wobei die Partikel der Form II in einer wässrigen Phase suspendiert vorliegen. Die Größe der Wirkstoffpartikel, d. h. die Größe, welche 90 Gew.-% der Wirkstoffpartikel nicht überschreiten, liegt dabei typischerweise unterhalb 30 µm, insbesondere unterhalb 20 µm. Vorteilhafterweise weisen in den erfindungsgemäßen SC's wenigstens 40 Gew.-% und insbesondere wenigstens 60 Gew.-% der Teilchen Durchmesser unterhalb 2 µm auf.

Wässrige Suspensionskonzentrate enthalten neben dem Wirkstoff typischerweise oberflächenaktive Substanzen, sowie gegebenenfalls Antischaummittel, Verdicker (= Andicker), Frostschutzmittel, Stabilisatoren (Biozide), Mittel zur Einstellung des pH-Wertes und Anticaking-Mittel.

Die Menge an Wirkstoff, d. h. die Gesamtmenge an Phenyluracil der Form II sowie an gegebenenfalls weiteren Wirkstoffen, liegt in derartigen SC's üblicherweise im Bereich von 10 bis 70 Gew.-%, insbesondere im Bereich von 20 bis 50 Gew.-%, bezogen auf das Gesamtgewicht des Suspensionskonzentrats.

Als oberflächenaktive Substanzen kommen vorzugsweise anionische und nichtionische Tenside in Betracht. Geeignete oberflächenaktive Substanzen sind auch Schutzkolloide. Die Menge an oberflächenaktiven Substanzen wird in der Regel 0,5 bis 30 Gew.-%, insbesondere 1 bis 20 Gew.-%, bezogen auf das Gesamtgewicht der erfindungsgemäßen wässrigen SC's betragen. Vorzugsweise umfassen die die oberflächenaktiven Substanzen wenigstens eine anionische oberflächenaktive Substanz und wenigstens eine nichtionische oberflächenaktive Substanz, wobei das Mengeverhältnis von anionischer zu nichtionischer oberflächenaktiver Substanz typischerweise im Bereich von 10 : 1 bis 1 : 10 liegt.

Zu den Beispielen anionischer oberflächenaktiver Substanzen (Tenside) zählen Alkylarylsulfonate, Phenylsulfonate, Alkylsulfate, Alkylsulfonate, Alkylethersulfate, Alkylarylethersulfate, Alkylpolyglykoletherphosphate, Polyarylphenyletherphosphate, Alkylsulfosuccinate, Olefinsulfonate, Paraffinsulfonate, Petroleumsulfonate, Tauride, Sarkoside, Fettsäuren, Alkylnaphthalinsulfonsäuren, Naphthalinsulfonsäuren, Ligninsulfonsäuren, Kondensationsprodukte sulfonierter Naphthaline mit Formaldehyd oder mit Formaldehyd und Phenol und gegebenenfalls Harnstoff sowie Kondensationsprodukte aus Phenolsulfonsäure, Formaldehyd und Harnstoff, Lignin-Sulfit-Ablauge und Ligninsulfonate, Alkylphosphate, Alkylarylphosphate, z. B. Tristyrylphosphate, sowie Polycarboxylate wie z. B. Polyacrylate, Maleinsäureanhydrid/Olefin-Copolymere (z. B. Sokalan^{®} CP9, BASF), einschließlich der Alkali-, Erdalkali-, Ammonium- und Amin-Salze der vorgenannten Substanzen. Bevorzugte anionische oberflächenaktive Substanzen sind solche, die wenigstens eine Sulfonatgruppe tragen und insbesondere deren Alkalimetall- und deren Ammoniumsalze.

Beispiele für nichtionische oberflächenaktiver Substanzen umfassen Alkylphenolalkoxylate, Alkoholalkoxylate, Fettaminalkoxylate, Polyoxyethylenglycerolfettsäureester, Rizinusölalkoxylate, Fettsäurealkoxylate, Fettsäureamidalkoxylate, Fettsäurepolydiethanolamide, Lanolineth-oxylate, Fettsäurepolyglykolester, Isotridecylalkohol, Fettsäureamide, Methylcellulose, Fettsäureester, Alkylpolyglykoside, Glycerolfettsäureester, Polyethylenglykol, Polypropylenglykol, Polyethylenglykolpolypropylenglykol-Blockcopolymere, Polyethylenglykolalkylether, Polypropylenglykolalkylether, Polyethylenglykolpolypropylenglykolether-Blockcopolymere (Polyethylenoxid-Polypropylenoxid-Blockcopolymere) und deren Gemische. Bevorzugte nichtionische oberflächenaktive Substanzen sind Fettalkoholethoxylate, Alkylpolyglykoside, Glycerolfettsäureester, Rizinusölalkoxylate, Fettsäurealkoxylate, Fettsäureamidalkoxylate, Lanolinethoxylate, Fettsäurepolyglykolester und Ethylenoxid-Propylenoxid-Blockcopolymere und deren Mischungen.

Schutzkolloide sind typischerweise wasserlösliche, amphiphile Polymere. Beispiele hierfür sind Proteine und denaturierte Proteine wie Casein, Polysaccharide wie wasserlösliche Stärkederivate und Cellulosederivate, insbesondere hydrophob modifizierte Stärken und Cellulosen, weiterhin Polycarboxylate wie Polyacrylsäure und Acrylsäurecopolymere, Polyvinylalkohol, Polyvinylpyrrolidon, Vinylpyrrolidon-Copolymere, Polyvinylamine, Polyethylenimine, und Polyalkylenether.

Für die erfindungsgemäßen wässrigen SC's geeignete, die Viskosität verändernde Additive (Andicker) sind insbesondere Verbindungen, die der Formulierung ein modifiziertes Fließverhalten verleihen, z.B. eine hohe Viskosität im Ruhezustand und niedrige Viskosität im bewegten Zustand. Geeignet sind grundsätzlich alle für diesen Zweck in Suspensionskonzentraten eingesetzte Verbindungen. Zu nennen sind beispielsweise anorganische Substanzen, z.B. Schichtsilikate und organisch modifizierte Schichtsilikate wie Bentonite oder Attapulgite (z. B. Attaclay^{®} Firma Engelhardt), und organische Substanzen wie Polysaccharide und Heteropolysaccharide wie Xanthan Gum^{®} (Kelzan^{®} der Fa. Kelco), Rhodopol^{®} 23 (Rhone Poulenc) oder Veegum^{®} (Firma R.T. Vanderbilt) zu nennen, wobei Xanthan-Gum^{®} bevorzugt verwendet wird. Die Menge der die Viskosität verändernden Additive beträgt häufig 0,1 bis 5 Gew.-%, bezogen auf das Gesamtgewicht des SC's.

Als für die erfindungsgemäßen wässrigen SC's geeignete Antischaummittel kommen beispielsweise für diesen Zweck bekannte Silikonemulsionen (Silikon^{®} SRE, Firma Wacker oder Rhodorsil^{®} der Firma Rhodia), langkettige Alkohole, Fettsäuren und deren Salze, Entschäumer vom Typ wässriger Wachsdispersionen, feste Entschäumer (sog. Compounds), fluororganische Verbindungen und deren Gemische in Betracht. Die Menge an Antischaummittel beträgt typischerweise 0,1 bis 1 Gew.-%, bezogen auf das Gesamtgewicht des SC's.

Den erfindungsgemäßen Suspensionskonzentraten kann man zur Stabilisierung auch Konservierungsmittel zusetzen. Geeignete Konservierungsmittel sind solche auf Basis von Isothiazolonen, beispielsweise Proxel^{®} der Fa. ICI oder Acticide^{®} RS der Fa. Thor Chemie oder Kathon^{®} MK der Firma Rohm & Haas. Die Menge an Konservierungsmittel beträgt typischerweise 0,05 bis 0,5 Gew.-%, bezogen auf das Gesamtgewicht des SC's.

Geeignete Frostschutzmittel sind flüssige Polyole, z. B. Ethylenglycol, Propylenglycol oder Glycerin sowie Harnstoff. Die Menge an Frostschutzmitteln beträgt in der Regel 1 bis 20 Gew.-%, insbesondere 5 bis 10 Gew.-%, bezogen auf das Gesamtgewicht des wässrigen Suspensionskonzentrats.

Gegebenenfalls können die erfindungsgemäßen wässrigen SC's Puffer zur pH-Wert Regulation enthalten. Beispiele für Puffer sind Alkalisalze schwacher anorganischer oder organischer Säuren wie z. B. Phosphorsäure, Borsäure, Essigsäure, Propionsäure, Citronensäure, Fumarsäure, Weinsäure, Oxalsäure und Bernsteinsäure.

Sofern die Formulierungen der kristallinen Modifikationen Form II zur Saatgutbehandlung eingesetzt werden, können sie weitere übliche Bestandteile enthalten, wie sie bei der Saatgutbehandlung, z. B. bei Beizen oder Coaten eingesetzt werden. Hierzu zählen neben den vorgenannten Bestandteilen insbesondere Farbmittel, Kleber, Füllstoffe und Weichmacher.

Als Farbmittel kommen alle die für derartige Zwecke üblichen Farbstoffe und Pigmente in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C. I. Pigment Red 112 und C. I. Solvent Red 1, Pigment blue 15:4, Pigment blue 15:3, Pigment blue 15:2, Pigment blue 15:1, Pigment blue 80, Pigment yellow 1, Pigment yellow 13, Pigment red 48:2, Pigment red 48:1, Pigment red 57:1, Pigment red 53:1, Pigment orange 43, Pigment orange 34, Pigment orange 5, Pigment green 36, Pigment green 7, Pigment white 6, Pigment brown 25, Basic violet 10, Basic violet 49, Acid red 51, Acid red 52, Acid red 14, Acid blue 9, Acid yellow 23, Basic red 10, Basic red 108 bekannten Farbstoffe und Pigmente. Die Menge an Farbmittel wird üblicherweise nicht mehr als 20 Gew.-% der Formulierung ausmachen und liegt vorzugsweise im Bereich von 0,1 bis 15 Gew.-%, bezogen auf das Gesamtgewicht der Formulierung.

Als Kleber kommen alle üblichen in Beizmitteln einsetzbaren Bindemittel in Frage. Beispiele für geeignete Bindemittel umfassen thermoplastische Polymere wie Polyvinylpyrrolidon, Polyvinylacetat, Polyvinylalkohol und Tylose weiterhin Polyacrylate, Polymethacrylate, Polybutene, Polyisobutene, Polystyrol, Polyethylenamine, Polyethylenamide, die vorgenannten Schutzkolloide, Polyester, Polyetherester, Polyanhydride, Polyesterurethane, Polyesteramide, thermoplastische Polysaccharide, z. B. Cellulosederivate wie Celluloseester, Celluloseether, Celluloseetherester, einschließlich Methylcellulose, Ethylcellullose, Hydroxymethylcellulose, Carboxymethylcellulose, Hydroxypropylcellulose und Stärkederivate und modifizierte Stärken, Dextrine, Maltodextrine, Alginate und Chitosane, weiterhin Fette, Öle, Proteine, einschließlich Casein, Gelatine und Zein, Gummi-Arabicum, Schellack. Vorzugsweise sind die Kleber pflanzenverträglich, d.h. sie weisen keine oder keinen nennenswerten phytotoxischen Wirkungen auf. Vorzugsweise sind die Kleber biologisch abbaubar. Vorzugsweise ist der Kleber so gewählt, dass er als Matrix für die aktiven Bestandteile der Formulierung wirkt. Die Menge an Kleber wird üblicherweise nicht mehr als 40 Gew.-% der Formulierung ausmachen und liegt vorzugsweise im Bereich von 1 bis 40 Gew.-% und insbesondere im Bereich von 5 bis 30 Gew.-%, bezogen auf das Gesamtgewicht der Formulierung.

Neben dem Kleber kann die Formulierung für die Saatgutbehandlung auch inerte Füllstoffe enthalten. Beispiele hierfür sind die vorgenannten festen Trägermaterialien, insbesondere feinteilige anorganische Materialien wie Tone, Kreide, Bentonit, Kaolin, Talkum, Perlit, Mica, Kieselgel, Diatomeenerde, Quarzpulver, Montmorillonit, aber auch feinteilige organische Materialen, wie Holzmehl, Getreidemehl, Aktivkohle und dergleichen. Die Menge an Füllstoff wird vorzugsweise so gewählt, dass die Gesamtmenge an Füllstoff 75 Gew.-%, bezogen auf das Gesamtgewicht aller nicht flüchtigen Bestandteile der Formulierung nicht überschreitet. Häufig liegt die Menge an Füllstoff im Bereich von 1 bis 50 Gew.-%, bezogen auf das Gesamtgewicht aller nicht flüchtigen Bestandteile der Formulierung.

Daneben kann die Formulierung für die Saatgutbehandlung noch einen Weichmacher enthalten, der die Flexibilität der Beschichtung erhöht. Beispiele für Weichmacher sind oligomere Polyalkylenglykole, Glycerin, Dialkylphthalate, Alkylbenzylphthalate, Glykolbenzoate und vergleichbare Verbindungen. Die Menge an Weichmacher in der Beschichtung liegt häufig im Bereich von 0,1 bis 20 Gew.-%, bezogen auf das Gesamtgewicht aller nicht flüchtigen Bestandteile der Formulierung.

Die Erfindung betrifft insbesondere auch Mittel für den Pflanzenschutz in Form eines nicht-wässrigen Suspensionskonzentrats. Derartige Suspensionskonzentrate enthalten die Form II des Phenyluracils I in einer feinteiligen partikulären Form, wobei die Partikel der Form II in einer nicht-wässrigen Phase suspendiert vorliegen. Die Größe der Wirkstoffpartikel, d. h. die Größe, welche 90 Gew.-% der Wirkstoffpartikel nicht überschreiten, liegt dabei typischerweise unterhalb 30 µm, insbesondere unterhalb 20 µm. Vorteilhafterweise weisen in den nicht-wässrigen SC's wenigstens 40 Gew.-% und insbesondere wenigstens 60 Gew.-% der Teilchen Durchmesser unterhalb 2 µm auf.

Nich-wässrige Suspensionskonzentrate enthalten neben dem Wirkstoff typischerweise oberflächenaktive Substanzen, sowie gegebenenfalls Antischaummittel, Verdicker (= Andicker), Frostschutzmittel, Stabilisatoren (Biozide), Mittel zur Einstellung des pH-Wertes und Anticaking-Mittel.

Die Menge an Wirkstoff, d.h. die Gesamtmenge an Phenyluracil I in der Form II sowie an gegebenenfalls weiteren Wirkstoffen, liegt in derartigen nicht-wässrigen SC's üblicherweise im Bereich von 10 bis 70 Gew.-%, insbesondere im Bereich von 20 bis 50 Gew.-%, bezogen auf das Gesamtgewicht des nicht-wässrigen Suspensionskonzentrats.

Als oberflächenaktive Substanzen kommen vorzugsweise die zuvor genannten anionischen und nichtionischen Tenside in Betracht. Die Menge an oberflächenaktiven Substanzen wird in der Regel 1 bis 30 Gew.-%, insbesondere 2 bis 20 Gew.-%, bezogen auf das Gesamtgewicht der erfindungsgemäßen nicht-wässrigen SC's betragen. Vorzugsweise umfassen die die oberflächenaktiven Substanzen wenigstens eine anionische oberflächenaktive Substanz und wenigstens eine nichtionische oberflächenaktive Substanz, wobei das Mengeverhältnis von anionischer zu nichtionischer oberflächenaktiver Substanz typischerweise im Bereich von 10:1 1 bis 1:10 liegt.

Die erfindungsgemäße Form I kann auch als Pulver-, Streu- und Stäubemittel formuliert werden. Derartige Formulierungen können durch Mischen oder gemeinsames Vermahlen der Form II mit einem festen Trägerstoff und gegebenenfalls weiteren Hilfsmitteln hergestellt werden.

Die erfindungsgemäße Form II kann auch in Form von Granulaten, z. B. Umhüllungs-, Imprägnierungs- und Homogengranulaten formuliert werden. Derartige Formulierungen können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nussschalenmehl, Cellulosepulver oder andere feste Trägerstoffe.

Die Konzentrationen der Form II in den anwendungsfertigen Zubereitungen können in weiten Bereichen variiert werden. Im Allgemeinen enthalten die Formulierungen etwa von 11 bis 98 Gew.-%, vorzugsweise 10 bis 95 Gew.-%, bezogen auf das Gesamtgewicht an Wirkstoffen.

Die folgenden Formulierungsbeispiele verdeutlichen die Herstellung solcher Zubereitungen:
I. 20 Gewichtsteile der Form II werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Auf diese weise erhält man ein wasserdispergierbares Pulver, welches die Form II enthält. Durch feines Verteilen der Mischung in 20000 Gewichtsteilen Wasser erhält man eine Spritzbrühe, die 0,1 Gew.-% der Form II enthält.
II. 3 Gewichtsteile der Form II werden mit 97 Gewichtsteilen feinteiligem Kaolin vermischt. Man erhält auf diese Weise ein Stäubemittel, das 3 Gew.-% der Form II enthält.
III. 20 Gewichtsteile der Form II werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenolsulfonsäure-Harnstoff-Formaldehyd-Kondensates und 68 Gewichtsteilen eines paraffinischen Mineralöls innig vermischt. Man erhält ein stabiles nichtwässriges Suspensionskonzentrat der Form II.
IV. 10 Gewichtsteile der Form II wurden in einer Lösung von 17 Gewichtsteilen eines Poly(ethylenglykol)(propylenglykol)blockcopolymeren, 2 Gewichtsteilen eines Phenolsulfonsäure-Formaldehyd-Kondensates und etwa 1 Gewichtsteil sonstigen Hilfsmitteln (Verdicker, Entschäumer) in einem Gemisch aus 7 Gewichtsteilen Propylenglykol und 63 Gewichtsteilen Wasser als Suspensionskonzentrat formuliert.

Die Applikation der Form II bzw. die der sie enthaltenden herbiziden Mittel erfolgt, sofern die Formulierung nicht bereits gebrauchsfertig ist, in Form wässriger Spritzbrühen. Diese werden durch Verdünnen der vorgenannten, die Form II des Phenyluracils I enthaltenden Formulierungen mit Wasser bereitet. Die Spritzbrühen können auch weitere Bestandteile in gelöster, emulgierter oder suspendierter Form enthalten, beispielsweise Düngemittel, Wirkstoffe anderer herbizider oder wachstumsregulierender Wirkstoffgruppen, weitere Wirkstoffe, z. B. Wirkstoffe zur Bekämpfung von tierischen Schädlingen oder phytopathogenen Pilzen bzw. Bakterien, weiterhin Mineralsalze, welche zur Behebung von Ernährungs- und Spurenelementmängeln eingesetzt werden, sowie nicht-phytotoxische Öle und Ölkonzentrate. In der Regel werden diese Bestandteile vor während oder nach dem Verdünnen der erfindungsgemäßen Formulierungen der Spritzbrühe zugesetzt.

Die Applikation der Form II bzw. die der sie enthaltenden herbiziden Mittel kann im Vorauflauf- oder im Nachauflaufverfahren erfolgen. Sofern das Phenyluracil I für gewisse Kulturpflanzen weniger verträglich ist, können Ausbringungstechniken angewandt werden, bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an der Form II betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte oder zur Selektivitätssteigerung kann die Form II mit zahlreichen Vertretern anderer herbizider oder wachstumsregulierender Wirkstoffgruppen und/oder Safenern gemischt und gemeinsam ausgebracht werden. Die Form II kann beispielsweise in Analogie zu den in WO2003/024221, WO 2004/080183, WO 2006/097509 und WO 2007/042447 beschriebenen Mischungen von Phenyluracilen I mit Herbiziden, Wachstumsregulatoren und/oder Safenern angewendet bzw. appliziert werden.

Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/- Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht. Geeignete Safener sind beispielsweise (Chinolin-8-oxy)essigsäuren, 1-Phenyl-5-haloalkyl-1 H-1,2,4-triazol-3-carbonsäuren, 1-Phenyl-4,5-dihydro-5-alkyl-1 H-pyrazol-3,5-dicarbonsäuren, 4,5-Dihydro-5,5-diaryl-3-isoxazolcarbonsäuren, Dichloroacetamide, alpha-Oximinophenylacetonitrile, Acetophenonoxime, 4,6-Dihalo-2-phenylpyrimidine, N-[[4-(Aminocarbonyl)phenyl]sulfonyl]-2-benzoesäureamide, 1,8-Naphthalsäureanhydrid, 2-Halo-4-(haloalkyl)-5-thiazolcarbonsäuren, Phosphorthiolate und N-Alkyl-O-phenylcarbamate sowie ihre landwirtschaftlich brauchbaren Salze, und vorausgesetzt sie haben eine Säurefunktion, ihre landwirtschaftlich brauchbaren Derivate, wie Amide, Ester und Thioester.

Außerdem kann es von Nutzen sein, die Form II allein oder in Kombination mit anderen Herbiziden und/oder Safenern auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nicht-phytotoxische Öle und Ölkonzentrate zugesetzt werden.

### Anwendungsbeispiele

Die herbizide Wirkung der Form II ließ sich durch die folgenden Gewächshausversuche zeigen:

Als Kulturgefäße dienten Kunststofftöpfe, die mit Boden (z. B. lehmiger Sand mit etwa 3,0 % Humus) als Substrat gefüllt wurden. Die Samen der Testpflanzen wurden nach Arten getrennt eingesät.

Bei Vorauflaufbehandlung wurden die in Wasser suspendierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 bis 25 °C bzw. 20 bis 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Nach den oben genanten Methoden wurden die erfindungsgemäße Form II und als Vergleichsverbindung die aus WO 01/83459 bekannte Form I, jeweils formuliert als wässriges Suspensions-Konzentrat (SC; 100 g/I) gegebenenfalls unter Zusatz von 1 I/ha Rustica Öl ® im Gewächshaustest verglichen. Die Suspensionskonzentrate wiesen folgende Zusammensetzung auf:

| | |
|---|---|
| Phenyluracil I | 100 g/L |
| 1,2-Propylenglykol | 70 g/L |
| Dispergiermittel I | 167 g/L |
| Dispergiermittel II | 20 g/L |
| Xanthan-Gum | 3 g/L |
| Biozid | 1,8 g/L |
| Wasser | ad 1 L |

| | |
|---|---|
| Dispergiermittel I: EO/PO-Blockcopolymer Dispergiermittel II: Phenolsulfonsäure-Formaldehyd-Kondensationsprodukt | |

Die in den Gewächshausversuchen verwendeten Pflanzen setzten sich aus folgenden Arten zusammen:

| Lateinischer Name | Deutscher Name |
|---|---|
| Ambrosia elatior | Beifußblättrige Ambrosia |
| Capsella bursa-pastoris | Gemeines Hirtentäschelkraut |
| Chenopodium album | Weißer Gänsefuß |
| Euphorbia heterophylla | Wolfsmilch |
| Galium aparine | Klebkraut |
| Glycine max | Sojabohne |
| Helianthus annuus | Sonnenblume |
| Hordeum vulgare | Wintergerste |
| Kochia scoparia | Besenradmelde |
| Lamium purpureum | purpurroteTaubnessel |
| Matricaria inodora | Geruchlose Kamille |
| Mercurialis annua | Einjähriges Bingelkraut |
| Papaver rhoeas | Klatschmohn |
| Pharbitis purpurea | Purpurtrichterwinde |
| Polygonum convolvulus | Windenknöterich |
| Salsola kali ssp. ruthenica | Russisches Salzkraut |
| Secale cereale | Winterroggen |
| Sida spinosa | Stachelige Samtmalve |
| Sinapis arvensis | Ackersenf |
| Stellaria media | Vogelsternmiere |
| Thlaspi arvense | Ackerhellerkraut |
| Triticum aestivum | Sommerweizen |
| Veronica persicaria | Persischer Ehrenpreis |
| Viola arvensis | Acherstiefmütterchen |

**Tabelle 4 Vergleich der herbiziden Wirksamkeit der Form II mit der aus WO 01/83459 bekannten Form I im Vorauflauf (Gewächshaus)**

| Testpflanzen | Aufwandmenge (g/ha a. S.) | Wirkstoff | |
|---|---|---|---|
| | | Form II | Form I |
| | | Schaden [%] | |
| Nutzpflanze: | | | |
| Glycine max | 25 | 30 | 70 |
| | 12,5 | 10 | 30 |
| | | | |
| Schadpflanze: | | | |
| Stellaria media | 25 | 100 | 75 |
| | 12,5 | 85 | 65 |
| Ambrosia elatior | 12,5 | 75 | 60 |
| Helianthus annuus | 12,5 | 100 | 70 |
| Euphorbia heterophylla | 12,5 | 100 | 95 |
| | 6,25 | 70 | 40 |
| Mercurialis annua | 6,25 | 100 | 40 |
| Pharbitis purpurea | 6,25 | 100 | 70 |
| Sida spinosa | 12,5 | 100 | 90 |

**Tabelle 5 Vergleich der herbiziden Wirksamkeit der Form II mit der aus WO 01/83459 bekannten Form I im Nachauflauf unter Zusatz von 1 I/ha Rustica Öl® (Gewächshaus)**

| Testpflanzen | Aufwandmenge (g/ha a. S.) | Wirkstoff | |
|---|---|---|---|
| | | Form II | Form I |
| | | Schaden [%] | |
| Nutzpflanze: | | | |
| Hordeum vulgare | 20 | 20 | 20 |
| | 15 | 10 | 15 |
| Secale cereale | 20 | 15 | 15 |
| | 15 | 10 | 10 |
| | 10 | 5 | 10 |
| Triticum aestivum | 20 | 15 | 15 |
| | | | |
| Schadpflanze: | | | |
| Capsella bursa-pastoris | 15 | 100 | 80 |
| Chenopodium album | 15 | 100 | 70 |
| Galium aparine | 15 | 100 | 75 |
| Lamium purpureum | 10 | 90 | 60 |
| Matricaria inodora | 5 | 100 | 65 |
| Thlaspi arvense | 5 | 100 | 70 |
| Polygonum convolvulus | 15 | 100 | 70 |
| Stellaria media | 5 | 100 | 50 |
| Viola arvensis | 5 | 90 | 40 |

**Tabelle 6 Vergleich der herbiziden Wirksamkeit der Form II mit der aus WO 01/83459 bekannten Form I im Nachauflauf (Gewächshaus)**

| Testpflanzen | Aufwandmenge (g/ha a. S.) | Wirkstoff | |
|---|---|---|---|
| | | Form II | Form I |
| | | Schaden [%] | |
| Nutzpflanze: | | | |
| Hordeum vulgare | 20 | 0 | 5 |
| | 15 | 0 | 5 |
| | | | |
| Schadpflanze: | | | |
| Kochia scoparia | 20 | 100 | 45 |
| Papaver rhoeas | 20 | 70 | 20 |
| Polygonum convolvulus | 20 | 100 | 40 |
| Salsola kali ssp. ruthenica | 20 | 100 | 80 |
| Sinapis arvensis | 10 | 80 | 50 |
| Thlaspi arvense | 10 | 98 | 30 |
| Veronica persicaria | 15 | 80 | 40 |

Die Testergebnisse zeigen deutlich, dass die erfindungsgemäße Form II bei gleicher oder besserer Toleranz durch die Nutzpflanze gegenüber der bekannten Form I eine deutlich verbesserte herbizide Wirksamkeit aufweist.

## Patentansprüche

1. Kristalline Form II von 2-Chlor-5-[3,6-dihydro-3-methyl-2,6-dioxo-4-(trifluormethyl)-1-(2H)-pyrimidinyl]-4-fluor-N-[[methyl-(1-methylethyl)amino]sulfonyl]-benzamid, worin der Anteil an Lösungsmittel im Kristallgitter unterhalb von 10 mol-% bezogen auf 2-Chlor-5-[3,6-dihydro-3-methyl-2,6-dioxo-4-(trifluormethyl)-1-(2H)-pyrimidinyl]-4-fluor-N-[[methyl-(1-methylethyl)amino]sulfonyl]benzamid liegt.

2. Kristallinen Form II gemäß Anspruch 1, die in einem Röntgenpulverdiffraktogramm bei 25 °C und Cu-Kα-Strahlung wenigstens zwei der folgenden, als 2θ-Werte angegebenen Reflexe zeigt: 6,3 ± 0,3°, 9,4 ± 0.3°, 10,9 ± 0,3°, 11,9 ± 0,3°, 12,6 ± 0,3°, 15,0 ± 0,3°, 15,8 ± 0,3°, 17,1 ± 0,3°, 20,0 ± 0.3°, 20,4 ± 0,3°, 24,7 ± 0,3°, 25,2 ± 0,3°, 26,2 ± 0,3°.

3. Kristalline Form II gemäß Anspruch 1 oder 2, die einen Schmelzpeak im Bereich von 170 bis 200°C mit einem Peakmaximum im Bereich von 180 bis 190°C.

4. Kristalline Form II gemäß einem der vorhergehenden Ansprüche mit einem Gehalt an 2-Chlor-5-[3,6-dihydro-3-methyl-2,6-dioxo-4-(trifluormethyl)-1-(2H)-pyrimidinyl]-4-fluor-N-[[methyl-(1-methylethyl)amino]sulfonyl]benzamidvon wenigstens 94 Gew.-%.

5. Verfahren zur Herstellung der kristallinen Form **II** gemäß einem der Ansprüche 1 bis 4, umfassend:
i) Bereitstellung einer Lösung von 2-Chlor-5-[3,6-dihydro-3-methyl-2,6-dioxo-4-(trifluormethyl)-1-(2H)-pyrimidinyl]-4-fluor-N-[[methyl-(1-methylethyl)-amino]sulfonyl]benzamid in einem organischen Lösungsmittel, worin die Konzentration an Wasser 10 Gew.-%, bezogen auf die Gesamtmenge an Lösungsmittel, nicht überschreitet,
ii) Bewirken einer Kristallisation von 2-Chlor-5-[3,6-dihydro-3-methyl-2,6-dioxo-4-(trifluormethyl)-1-(2H)-pyrimidinyl]-4-fluor-N-[[methyl-(1-methylethyl)amino]sulfonyl]benzamid über einen Zeitraum von wenigstens 1h.

6. Verfahren gemäß Anspruch 5, **dadurch gekennzeichnet, dass** das organische Lösungsmittel ausgewählt ist unter C₁-C₆-Alkanolen, acyclischen Ketonen mit 3 bis 8 C-Atomen, cyclischen Ketonen mit 5 bis 8 C-Atomen, Mono-C₁-C₃-Alkylbenzolen, Chlorbenzol, Dichlorbenzolen, Di-C₁-C₆-alkylethern, 5- oder 6-gliedrigen alicyclischen Ethern, Nitroalkanen mit 1 bis 3 C-Atomen, C₁-C₄-Alkylester aliphatischer C₁-C₄-Carbonsäuren, Alkylnitrilen mit 2 bis 6 C-Atomen, N,N-Dimethylamiden aliphatischer C₁-C₄-Carbonsäuren und deren Gemischen.

7. Verfahren gemäß einem der Ansprüche 5 oder 6, wobei man die Kristallisation durch Abkühlen und/oder Einengen der in Schritt 1 bereitgestellten Lösung bewirkt.

8. Verfahren gemäß einem der Ansprüche 5 bis 7, wobei man die Kristallisation durch Zugabe eines die Löslichkeit vermindernden Lösungsmittels bewirkt.

9. Verfahren gemäß einem der Ansprüche 5 bis 8, wobei die Kristallisation in Gegenwart von Impfkristallen der Form II erfolgt.

10. Pflanzenschutzmittel, enthaltend die kristalline Form II gemäß einem der Ansprüche 1 bis 4 und für die Formulierung von Pflanzenschutzmitteln übliche Hilfsmittel.

11. Pflanzenschutzmittel gemäß Anspruch 10 in Form eines wässrigen Suspensionskonzentrats.

12. Pflanzenschutzmittel gemäß Anspruch 10 in Form eines nicht-wässrigen Suspensionskonzentrats.

13. Verfahren zur Bekämpfung von unerwünschtem Pflanzenwuchs, **dadurch gekennzeichnet, dass** man die Form II nach einem der Ansprüche 1 bis 4 auf Pflanzen, deren Lebensraum und/oder auf Samen einwirken lässt.

## Claims

1. A crystalline form II of 2-chloro-5-[3,6-dihydro-3-methyl-2,6-dioxo-4-(trifluoromethyl)-1-(2H)-pyrimidinyl]-4-fluoro-N-[[methyl-(1-methylethyl)-amino]sulfonyl]benzamide, in which the amount of solvent in the crystal lattice is less than 10 mol% based on 2-chloro-5-[3,6-dihydro-3-methyl-2,6-dioxo-4-(trifluoromethyl)-1-(2H)-pyrimidinyl]-4-fluoro-N-[[methyl-(1-methylethyl)amino]sulfonyl]benzamide.

2. The crystalline form II according to claim 1 which, in an X-ray powder diffractogram at 25°C and Cu-K_{α} radiation, shows at least two of the following reflexes, given as 2θ values: 6.3 ± 0.3°, 9.4 ± 0.3°, 10.9 ± 0.3°, 11.9 ± 0.3°, 12.6 ± 0.3°, 15.0 ± 0.3°, 15.8 ± 0.3°, 17.1 ± 0.3°, 20.0 ± 0.3°, 20.4 ± 0.3°, 24.7 ± 0.3°, 25.2 ± 0.3°, 26.2 ± 0.3°.

3. The crystalline form II according to claim 1 or 2 which has a melting peak in the range of from 170 to 200°C with a peak maximum in the range of from 180 to 190°C.

4. The crystalline form II according to any of the preceding claims with a 2-chloro-5-[3,6-dihydro-3-methyl-2,6-dioxo-4-(trifluoromethyl)-1-(2H)-pyrimidinyl]-4-fluoro-N-[[methyl-(1-methylethyl)amino]sulfonyl]benzamide content of at least 94% by weight.

5. A process for the preparation of the crystalline form II according to any of claims 1 to 4, comprising:
i) the provision of a solution of 2-chloro-5-[3,6-dihydro-3-methyl-2,6-dioxo-4-(trifluoromethyl)-1-(2H)-pyrimidinyl]-4-fluoro-N-[[methyl-(1-methylethyl)amino]sulfonyl]benzamide in an organic solvent, in which the concentration of water does not exceed 10% by weight, based on the total amount of solvent,
ii) bringing about a crystallization of 2-chloro-5-[3,6-dihydro-3-methyl-2,6-dioxo-4-(trifluoromethyl)-1-(2H)-pyrimidinyl]-4-fluoro-N-[[methyl-(1-methylethyl)amino]sulfonyl]benzamide over a period of at least 1 h.

6. The process according to claim 5, wherein the organic solvent is selected among C₁-C₆-alkanols, acyclic ketones having 3 to 8 carbon atoms, cyclic ketones having 5 to 8 carbon atoms, mono-C₁-C₃-alkylbenzenes, chlorobenzene, dichlorobenzenes, di-C₁-C₆-alkyl ethers, 5- or 6-membered alicyclic ethers, nitroalkanes having 1 to 3 carbon atoms, C₁-C₄-alkyl esters of aliphatic C₁-C₄-carboxylic acids, alkylnitriles having 2 to 6 carbon atoms, N,N-dimethylamides of aliphatic C₁-C₄-carboxylic acids, and their mixtures.

7. The process according to claim 5 or 6, wherein the crystallization is brought about by cooling and/or concentrating the solution provided in step 1.

8. The process according to any of claims 5 to 7, wherein the crystallization is brought about by the addition of a solubility-reducing solvent.

9. The process according to any of claims 5 to 8, wherein the crystallization is effected in the presence of seed crystals of form II.

10. A plant protection composition comprising the crystalline form II according to any of claims 1 to 4 and auxiliaries conventionally employed for the formulation of plant protection compositions.

11. The plant protection composition according to claim 10 in the form of an aqueous suspension concentrate.

12. The plant protection composition according to claim 10 in the form of a nonaqueous suspension concentrate.

13. A method of controlling undesired vegetation, wherein form II according to any of claims 1 to 4 is allowed to act on plants, their environment and/or on seeds.

## Revendications

1. Forme cristalline II de 2-chloro-5-[3,6-dihydro-3-méthyl-2,6-dioxo-4-(trifluorométhyl)-1-(2H)-pyrimidinyl]-4-fluoro-N-[[méthyl-(1-méthyléthyl)amino]sulfonyl]-benzamide, dans laquelle la proportion de solvant dans le réseau cristallin est inférieure à 10% en moles par rapport au 2-chloro-5-[3,6-dihydro-3-méthyl-2,6-dioxo-4-(trifluorométhyl)-1-(2H)-pyrimidinyl]-4-fluoro-N-[[méthyl-(1-méthyléthyl)amino]sulfonyl]-benzamide.

2. Forme cristalline II selon la revendication 1, qui présente sur un diffractogramme de rayons X sur poudre à 25°C et avec un rayonnement Cu-Kα au moins deux des réflexions suivantes, données sous la forme de valeurs 2θ : 6,3 ± 0,3°, 9,4 ± 0,3°, 10,9 ± 0,3°, 11,9 ± 0,3°, 12,6 ± 0,3°, 15,0 ± 0,3°, 15,8 ± 0,3°, 17,1 ± 0,3°, 20,0 ± 0,3°, 20,4 ± 0,3°, 24,7 ± 0,3°, 25,2 ± 0,3°, 26,2 ± 0,3°.

3. Forme cristalline II selon la revendication 1 ou 2, qui présente un pic de fusion dans la plage allant de 170 à 200°C avec un maximum de pic dans la plage allant de 180 à 190 °C.

4. Forme cristalline II selon l'une quelconque des revendications précédentes, ayant une teneur en 2-chloro-5-[3,6-dihydro-3-méthyl-2,6-dioxo-4-(trifluorométhyl)-1-(2H)-pyrimidinyl]-4-fluoro-N-[[méthyl-(1-méthyléthyl)amino]sulfonyl]-benzamide d'au moins 94 % en poids.

5. Procédé de fabrication de la forme cristalline II selon l'une quelconque des revendications 1 à 4, comprenant :
i) la préparation d'une solution de 2-chloro-5-[3,6-dihydro-3-méthyl-2,6-dioxo-4-(trifluorométhyl)-1-(2H)-pyrimidinyl]-4-fluoro-N-[[méthyl-(1-méthyléthyl)amino]sulfonyl]-benzamide dans un solvant organique, la concentration d'eau ne dépassant pas 10 % en poids par rapport à la quantité totale de solvant,
ii) la provocation d'une cristallisation de 2-chloro-5-[3,6-dihydro-3-méthyl-2,6-dioxo-4-(trifluorométhyl)-1-(2H)-pyrimidinyl]-4-fluoro-N-[[méthyl-(1-méthyléthyl)amino]sulfonyl]-benzamide pendant une durée d'au moins 1 h.

6. Procédé selon la revendication 5, **caractérisé en ce que** le solvant organique est choisi parmi les alcanols en C₁-C₆, les cétones acycliques de 3 à 8 atomes C, les cétones cycliques de 5 à 8 atomes C, les monoalkylbenzènes en C₁-C₃, le chlorobenzène, les dichlorobenzènes, les éthers de dialkyle en C₁-C₆, les éthers alicycliques à 5 ou 6 éléments, les nitroalcanes de 1 à 3 atomes C, les esters alkyliques en C₁-C₄ d'acides carboxyliques aliphatiques en C₁-C₄, les alkylnitriles de 2 à 6 atomes C, les N,N-diméthylamides d'acides carboxyliques aliphatiques en C₁-C₄ et leurs mélanges.

7. Procédé selon l'une quelconque des revendications 5 ou 6, dans lequel la cristallisation est provoquée par refroidissement et/ou concentration de la solution préparée à l'étape 1.

8. Procédé selon l'une quelconque des revendications 5 à 7, dans lequel la cristallisation est provoquée par ajout d'un solvant réduisant la solubilité.

9. Procédé selon l'une quelconque des revendications 5 à 8, dans lequel la cristallisation a lieu en présence de cristaux d'inoculation de la forme II.

10. Agent phytosanitaire, contenant la forme cristalline II selon l'une quelconque des revendications 1 à 4 et des adjuvants usuels pour la formulation d'agents phytosanitaires.

11. Agent phytosanitaire selon la revendication 10, sous la forme d'un concentré de suspension aqueux.

12. Agent phytosanitaire selon la revendication 10, sous la forme d'un concentré de suspension non aqueux.

13. Procédé de lutte contre une végétation indésirable, **caractérisé en ce que** la forme II selon l'une quelconque des revendications 1 à 4 est laissée agir sur des plantes, leur habitat et/ou sur des semences.
